# EUROPEAN PATENT APPLICATION

(11) **EP 1 176 208 A1**
(43) Date of publication of application: **30.01.2002**
(21) Application number: 00116451.6
(22) Date of filing: 28.07.2000
(51) Int. Cl.: C12P 17/06, C12P 7/42, C12N 1/14, C12P 1/02, A23L 1/20, A61K 35/70, A61K 31/365

(54) **Koji molds and use thereof for preparing cholesterol-lowering products**

(71) Applicant: Société des Produits Nestlé S.A., 1800 Vevey (CH)
(72) Inventor: Hajjaj, Hassan, 1073 Savigny (CH); Van den Broek, Peter, 1066 Epalinges (CH); Niederberger, Peter, 1066 Epalinges (CH); Fay, Laurent Bernard, 74500 Evian (FR); Mace, Katherine, 1095 Lutry (CH); Neeser, Jean-Richard, 1073 Savigny (CH)
(74) Representative: Vuille, Roman

(57) **Abstract**

The invention concerns a naturally occuring koji mold wich is incapable of producing a toxin and wich produces at least one compound wich is capable of lowering serum cholesterol concentration for use in a fermented food product. The invention also deals with the use of such mold in the manufacture of a food product.

Process for the production of a fermented food product exhibiting cholesterol-lowering properties by inoculating crop material with a mold according to the invention.

Food product with cholesterol-lowering properties containing compound stemming from a mold according to the invention.

## Description

The present invention relates to micro-organisms which produce cholesterol lowering compounds and the use of these micro-organisms in the production of fermented food products such as bio-hydrolysates, soya sauces or seasonings, for example with cholesterol lowering properties.
Biohydrolysates like soya sauces are traditionally produced through a two or three steps process : a first step of Koji production. In this first step, cooked Soya beans or defatted soya flour are mixed with roasted wheat, the mixture is inoculated with a culture of Koji mold, cultured under aerobiosis and intermittent stirring for one to four days to give a Koji. A second step consists of preparation of a Moromi by addition of water and salt to initiate hydrolysis. This Moromi is left to ferment with Moromi yeasts for about 6 to 8 months. A final step of isolating liquid sauce from solids is finally realised.

In the context of the invention, the term "Koji" designates the product of fermentation, with a Koji culture, of a mixture of a protein source and a carbohydrate source, more particularly a mixture of a cooked pulse or oilseed and a cooked or roasted cereal, for example a mixture of cooked Soya or haricot bean and cooked or roasted wheat or rice.
In the context of the invention, a Koji culture is understood to be a culture or Koji spores of the type available on the market, wich in particular comprises spores of "yellow Aspergilli".

Fermenting any other source of protein can make other kind of biohydrolysates. Thus materials, preferentially containing glutamic acid-rich proteins, such as for example oilseed cakes, pulses or cereal gluten, are widely used in hydrolyzed form as a starting material in the composition of dehydrated or liquid soups, sauces and seasonings.

This kind biohydrolysates which can be used to prepare liquid seasoning are very appreciated because they show strong and rich aromatic taste and are therefore used as seasoning in various meals. These hydrolysates can also be useful as aromatic bases for food or flavouring industries because of their great aromatic profile.

Nowadays the nutritional awareness leads the food industry to provide food stuffs not only with optimal organoleptic features but also with improved nutritional functionalities. The importance of excessive serum cholesterol levels as a main risk factor for the occurrence of atherosclerosis of the coronary and peripheral vasculature is generally recognized. Extensive clinical studies have led to the conclusion that the risk of suffering from coronary diseases can be decreased by reduction of the serum cholesterol level, and in particular of "Low density lipoprotein" levels. Since the major proportion of the cholesterol in the body is derived via de-novo biosysnthesis in the liver and only a smaller part is usually derived by absorption from the food, the inhibition of cholesterol-biosynthesis represents a particularly attractive method of lowering increased plasma cholesterol levels.

In Asian countries for example, several fermented products are used for their health beneficial properties. These food products are often derived from molds fermenting plant raw materials. A good example is Chinese "Red-Yeast-Rice", a rice fermented with a mold of the genus Monascus. This product contains metabolites of the class of Monacolins that has been demonstrated to have a cholesterol-lowering effect. These Monacolins belonging to the "statin family" of compounds act as inhibitors of an enzyme in cholesterol biosysnthesis called HMG-CoA-reductase reponsible of the conversion of HMG-CoA to mevalonate. Statins are successfully applied, in pure form in pharmaceutical products with the main action to lower LDL-cholesterol level (Low Density Lipoproteincholesterol). As a matter of fact HMGCoAreductase acts as a key enzyme in the biosynthesis of the cholesterol. Thus, the product MEVACOR® from MERCK, for example, contains isolated and purified Lovastatin from the filamentous mold *Aspergillus terreus.* This compound is also related to the statin family and shows inhibitory activity against HMG-CoA-reductase. On the other hand, PHARMANEX brought a "dietary supplement" Cholestin® on the market that contains molecules of the Monacolin family, and in which the active metabolites are produced by the filamentous mold *Monascus purpureus.*
However, it has been shown that *Monascus purpureus* is potentially able to produce a nephrotoxic and/or mutagenic compound called citrinin (Sabater-Vilar M, Maas RF and Fink-Gremmels J. *Mutat. Res.* 1999 Jul 21 ; 444(1): 7-16). For this reason the status of the dietary supplement Cholestine and, all the more of any potential food products, containing this mold are under strong debate in the USA and in European countries from a legal, safety and toxicological point of view. The same problem may arise with *Aspergillus terreus* that is known to have the potential to produce Citrinin.
Therefore *Aspergillus terreus* or *Monascus pupureus* are not micro-organisms which are applicable in food processes or directly in food products. Thus it is known that several filamentous molds can produce compounds belonging to the statin family, but such molds are not "food-grade" because of their potential to produce toxins and for this reason cannot be used in food fermentation processes and related food products.
EP 556699 (NOVOPHARM) Describes the transformation of non lovastatin producing Aspergillus strains *(Aspergillus oryzae)* to lovastatin-producing strains by introduction of *Aspergillus terreus* genomic DNA. This patent teaches the use of genetic modification as a means of obtaining statinproducing filamentous molds, which have a "food-grade" status. The filamentous molds of the claimed species *Aspergillus oryzae* are therefore not of "natural" but of recombinant origin.

The aim of the present invention is to provide natural food-grade micro-organisms for application in a fermented food with cholesterol-lowering properties.

As natural or naturally occuring micro-organisms, it is understood a micro-organism that has not been genetically modified and can be selected from the environment.

To this end, the micro-organism according the invention is an isolated naturally occuring micro-organism which is incapable of producing a toxin and wich produces at least one compound wich is capable of lowering serum cholesterol concentration.
It is an object of the invention to provide a naturally occuring micro-organism wich is incapable of producing a toxin and wich produces at least one compound wich is capable of lowering serum cholesterol concentration for use in a fermented food product.

As incapable of producing a toxin, it is understood that the micro-organism has not the capability to produce a toxin whatever the environmental and/or growth conditions may be.

The present invention also concerns a process for the production of a fermented food such as a protein hydrolysate, for example, wich can comprises the steps of inoculating protein containing material with a micro-organism according to the invention to effect Koji, adding water to the resulting preparation and hydrolysing the resulting preparation to obtain the hydrolysate.

Finally, the present invention also concerns the use in a fermented food product of at least one compound stemming from a micro-organism according to the invention in the manufacture of a food product or medicament for modulation of serum cholesterol levels or treatment of cholesterol related deseases.
It is thus an object of the invention to provide a food product with serum cholesterol-lowering properties characterized in that it contains at least one compound stemming from a micro-organism according to the invention.

In the present specification, with the term « fermented food product », it should be understood an edible product whose production process comprises at least one fermentation step involving the use of at least a micro-organism according to the invention. Furthermore, with the term "edible product", it should also be understood a product whose consumption does not have toxic nor harmful secondary effects on human health. In particular, due to the presence of cholesterol-lowering compounds, such "fermented food product" can provide health-beneficial functionality. Indeed, the micro-organism according to the invention can be selected among micro-organisms that exhibit safe-history of use for human consumption.
It is noteworthy to notice that the cholesterol lowering effect obtained thank to the use of a micro-organism according to the invention is achieved by inhibiting cholesterol biosynthesis.
In a particular embodiment of the invention, the cholesterol lowering effect is achieved by inhibition of HMG-CoA-reductase. Indeed, one of the cholesterol-lowering compounds produced by a micro-organism according the invention which is capable of lowering cholesterol concentration through HMG-CoA-reductase inhibition is lovastatin.
However, a micro-organism according to the invention can produce at least one cholesterol-lowering compound that is not lovastatin and that can also inhibit cholesterol biosynthesis. HMG-CoA-reductase catalyses the conversion of HMG-CoA to mevalonate, but this step is just one step in the whole cholesterol biosynthesis chain. At least one of the compounds produced by a micro-organism according to the invention inhibits at least one step downstream of mevalonate. Such downstream inhibition can be observed by adding mevalonate to bypass the HMG-COA/ Mevalonate step.
One of the key features of the micro-organism according to the invention is that it is incapable of producing a toxin, particularly incapable of producing an aflatoxin.
The said micro-organism capable of producing at least one cholesterol-lowering compound, without any production of toxin may be a micro-organism used in the preparation of the product, for example, particularly a filamentous mold such as Monascus, Penicillium and more preferentially molds belonging to the genus Aspergillus, for example, providing that the micro-organism be selected for its ability to synthetize cholesterol-lowering compounds, but incapable to produce a toxin. Then, this micro-organism may be selected among the broad variety of micro-organisms having a safe history of use in human consumption and usable for food production or in a food product it-self.
In a preferred embodiment of the present process, the food product made through the use of a micro-organism according to the present invention may be a liquid seasoning, such as a soya sauce, or a seasoning paste or powder, for example.
The micro-organism according to the invention can be reduced to practice in a traditionnal soya sauce or seasoning production process. It can also be used, alone or in combination with other Koji molds in any process such as the ones described in EP 0429760 (process for preparing a flavouring agent), EP 0829205 (seasoning product) or EP 0824 873 (production of seasoning).

It is also an object of the invention to use a micro-organism according to the present invention in the manufacture of a fermented food product or medicament for modulation of serum cholesterol levels or treatment of cholesterol related deseases.
It is an other object of the invention to use at least one compound stemming from a micro-organism according to the present invention in the manufacture of a food product or medicament for modulation of serum cholesterol levels or treatment of cholesterol related deseases.
Among the different steps of the process, a characteristic one is the fermentation of a plant material with at least one micro-organism according to the invention, for example, or with a combination of at least one micro-organism according to the invention and a traditional fermenting micro-organism that does not produce cholestrol-lowering compound, for example. This first Koji step may also be conducted in the presence of added proteolytic enzymes from various origins, for example.

In a preferred embodiment where the process is related to the manufacturing of a liquid seasoning such as Soya sauce, the said micro-organism is a mold, preferentially a Koji mold belonging to the genus Aspergillus, for example, that has been selectively screened and isolated among the wide variety of existing Koji mold strains for its ability to produce at least one cholesterol-lowering compound but its lack of a potential to produce toxin. To this end and for this preferred embodiment, 12 strains of the genus *Aspergillus* have been deposited, by way of example, according Budapest Treaty, on 06.14.2000 and on 07.27.2000, at CNCM, collection Nationale de Culture de Micro-organismes, Institut Pasteur, Rue du Dr Roux, 75724 Paris, France, where they received the N° CNCM 1-2489, CNCM I-2490, CNCM I-2525, CNCM I-2526, CNCM I-2527, CNCM I-2528, CNCM I-2529, CNCM I-2530, CNCM I-2531, CNCM I-2532, CNCM I-2533 and CNCM I-2534.

In such a preferred embodiment, the protein containing material may be a plant material such as wheat grains, soya, rice, maize, oil seeds, or fractions of plant materials such as wheat gluten, wheat bran, for example or any mixture of such plant materials or plant material fractions, for example. The different steps of the process of soya sauce production can be done according to traditional know-how of the man skilled in the art of the manufacturing of fermented sauces such as soya sauces. The main feature is the use or the addition of micro-organisms according to the invention, preferentially Koji molds.

The soya material may be cooked by soaking crushed soya beans, i.e., soya meal, for a few hours and then subjecting the meal to a temperature of approximately 120°C to 140 °C for a few minutes, for example. The cooked soya meal may be mixed with crushed roasted grains of wheat, i.e., a roasted wheat meal, for example.

After cooling, the mixture may be inoculated with spores of the Koji mold according to the invention or a mix of these mold spores and traditional ones from commercial suppliers, for example. The mixture may then be left to ferment on a tray or in a commercial apparatus specially designed for this purpose with intermittent stirring and constant aeration. This fermentation step promotes the production of proteases by the Koji molds present in the mix and in particular at least one compound wich is capable of lowering cholesterol concentration by the molds according to the invention that are present in this mix.

The Koji mix may be suspended by mixing with water, for example. The hydrolysis step can be carried out for several hours at 30°C to 60°C, for example.

The subsequent steps can be carried out according classical process known by the man skilled in the art. Thus the Moromi step may be realised by addition of salt and a traditional Moromi yeast of the species *Candida versatilis* or *Saccharomyces rouxii,* for example. The moromi thus inoculated may be left to ferment from days to months with stirring and aeration, for example.

The Moromi may then be pressed, for example, in a filter press, the insolubles may be removed and the liquor obtained may be pasteurised.

Accordingly, it is possible by this particular process to obtain a fermented liquid seasoning such as this soya sauce comparable in taste and aroma with a fermented soya sauce obtained by a traditional process. The principal feature of this soya sauce, beyond its aroma, is its health beneficial functionality due to the presence of at least one cholesterol-lowering compound contained in it but without trace of any toxin such as aflatoxin, for example.

Thus, this fermented liquid seasoning can be used either as a seasoning and/or as a cholesterol-lowering dietary supplement, for example.

The micro-organism according to the invention, which can be any food micro-organism, notably used for the preparation of the fermented food product, is capable of producing at least one cholesterol-lowering compound but is lacking the ability to produce a toxin and therefore is usable in the manufacturing of any fermented food-stuffs production. The micro-organism according to the invention can be filamentous molds present or used for production of fermented aromatised products (sake, soya sauce, vegetable seasoning, peanut sauce, for example) providing that the micro-organism be selected and isolated among all the natural existing micro-organisms for its ability to produce at least one cholesterol-lowering compound but no toxin.

Thus, in order to select and identify micro-organisms that respond to the previous conditions, different kind of micro-organisms from different sources can be checked and submitted to one selection method such as the one described in the example below, for example. The example below is given as illustration of a selection method for identification of a micro-organism according to the invention. This specific selection method comprises the steps of culturing micro-organisms on a growth medium, preparing and purifying the culture crude extracts and submitting the latter to lovastatin and toxin analysis and to a test measuring cholesterol synthesis inhibition. Such a method can be applied to any kind of micro-organism in order to check if it satisfies the desired criteria. Furthermore, any other equivalent method for cultivating micro-organisms, isolating and detecting lovastatin and/or toxin, evaluating influence of the fermentative extracts on cholesterol biosynthesis can be used in order to identify the micro-organism that fulfils the criteria and corresponds to the features of the micro-organism according to the invention.
A method for selecting micro-organism according to the invention is described in the following examples. This method is not limited to the micro-organisms screened. The toxin detection and identification method is presently directed to aflatoxins class compounds but can be modified and adapted for any kind of toxic compounds.

### EXEMPLE 1

### Micro-organism growth conditions :

Approximately 10⁷ conidiospores of micro-organism to test were used to inoculate 40 milliliters (ml) of medium A in a 250 ml unbaffled Erlenmeyer flask for primary seed culture. The seed medium (medium A) contained per litre : 10 g of glucose, 5 g of corn steep liquor, 40 g of tomato paste, 10 g of Oat meal, and trace elements : 1g FeSo₄.7H₂O, 1g MnSO₄.4H₂O, 200 mg ZnSO₄.7H₂O, 100 mg CaCl₂.2H₂O, 25 mg CuCl₂.2H₂O, 56 mg H₃Bo₃, and 19 mg (NH₄)₆Mo₇O₂₄.4 H₂O. These cultures were incubated for 24 hours at 30°C in an orbital shaker at 200 rpm.
Secondary cultures were prepared by adding 6 ml of the primary seed culture to a 1 1 unbaffled Erlenmeyer flask containing 200 ml of production medium (B).

The production medium (B) contained per liter: 45 g of glucose or lactose, 24 g of peptonized milk, 2.5 g of yeast extract, and 2.5 g of polyethylene glycol P2000 (pH 6.5). The flasks were shaken in an orbital shaker at 200 rpm at 28°C for 12 days.

### Extraction and purification :

After fermentation (280 h), the culture (200 ml) was macerated with 80 % methanol (200 ml) for 2 hours. The mixture was filtered through a filter paper and the filtrate was evaporated. The crude extract was recovered with water and this solution was acidified and adjusted to pH 3 with 3 N hydrochloric acid (HCl).
The aqueous extract was extracted several times with ethyl acetate (v-v). The organic phase was dried with anhydrous Na₂SO₄ and evaporated in vacuum (30°C) to remove the organic solvent. The residue was taken up in 10 ml MeOH for analysis chromatography (HPLC and TLC), spectroscopy (LC, MS and LC/MS/MS) and *in-vitro* test (inhibition of the enzyme 3-hydroxy-3-methylglutaryl-coenzyme A reductase [HMG-CoA reductase ; mevalonate : NADP⁺ oxidoreductase (CoA-acylating), EC 1.1.1.34]) to identify and quantify Lovastatin. The production of aflatoxin was evaluated by HPLC.

### Lovastatin analysis :

The lovastatin was determined by high-performance liquid chromatography (HPLC) and mass spectroscopy.
HPLC ANALYSIS: nucleosil 100-5 C₁₈ column (250 x 4 mm) (Macherey & Nagel) was used with a post column (Lichrospher 100 RP-18 (Merck). Solvent A: 0.05 % H₃PO₄ in water, solvent B was acetonitrile. The separation started with a linear gradient from 95 % A and 5 % B, reaching 50 % A and 50 % B in 45 min, the 30 % A and 70 % B in 46 min, then 10 % A and 90 % in 48 min, then 0 % A and 100 % B in 50 min and continued with an isocratic run for 4 minutes. Initial conditions were maintained for 6 minutes to re-equilibrate the column. The flow rate was 1 ml/min. The detector used was Hewlett Packard G1315 A, serie 1100, the wave length detection was at λₘₐₓ = 254 nm.
HPLC/MS and HPLC/MS/MS ANALYSIS : Separation was performed using a Waters HPLC system, consisting of a type 757 autosampler, a 600-MS pump with system controller and a type 486-MS UV-detector. The UV absorption at 258 nm was recorded using an analogue input to the mass spectrometer's data system. A Nucleosil 100-C18 HPLC column (250 mm x 4 mm I.D., Macherey & Nagel) was used with a post column splitter 1/10 before the mass spectrometer. Solvent A was 0.1 % trifluoroacetic acid in water, solvent B was acetonitrile. Using a flow rate of 1mL/min the separation started with a linear gradient from 95 % A and 5 % B, reaching 50% A and 50 % B in 30 min, then 30% A and 70 % B in 31 min, then 10 % A and 90 % B in 33 min, then 0 % A and 100 % B in 35 min and continued with an isocratic run for 5 minutes. Initial conditions were reached within 5 minutes and were maintained for 5 minutes to re-equilibrate the column.
The mass spectrometer was a Finnigan TSQ 700 triple quadrupole mass spectrometer (San Jose, CA, USA) equipped with an electrospray ionization source. Data acquisition was performed on a DECstation 2100 running under Ultrix 4.2A (Digital Equipment, USA) using the Finnigan software package ICIS2, Version 7.0. The transfer capillary was set at 200 °C and the spray at 4.2 kV. Full scan mass spectra were acquired in positive mode by scanning from *m/z* 50 to *m/z* 600 in 1 second. Daughter ion spectra were obtained from *m/z* 20 to *m/z* 450 at a collision energy of -15 eV in the laboratory frame using Argon at a pressure of 1 mTorr as the collision gas. Lovastatin was detected using the same conditions after selective reaction monitoring of the daughter ions at *m/z* 199, 285 and *m/z* 303 from the protonated parent ion at *m/z* 405. Detection by LC/MS/MS of these closely structure-related daughter ions allows the specific determination of Lovastatin in the samples.

### Aflatoxin analysis :

The concentration of individual aflatoxins in methanolic extracts was measured by isocratic HPLC using postcolumn derivatization with on-line electrochemically-generated bromine and fluorescence detection. A reversed-phase ODS Hypersil column (3 µm, 125 mm x 4.6 mm i.d.) was used along with an ODS Hypersil guard column (3 µm, 25 mm x 4.6 mm i.d.), both from Metrohm-Bischoff AG (Leonberg, Germany). The mobile phase consisted of a mixture of water/acetonitrile/methanol (60:5:35) v/v/v, containing 119 mg potassium bromide and 100 µl 65% nitric acid per liter. Elution was carried out at a flow rate of 1.0 ml/min and at room temperature. The postcolumn derivatization system consisted of a KOBRA cell with variable control current source (Rhône Diagnostics Technologies Ltd, Glasgow, Scotland). Current setting was adjusted at 100 µA. The individual aflatoxins were monitored with a Waters model 470 scanning fluorescence detector (excitation wavelength 365 nm, emission wavelength 428 nm).

The presence of aflatoxins in positive culture supernatants was confirmed by using a more selective method based on an immunoaffinity column cleanup, according to the procedure described by Trucksess et al. (1991). 1 ml aliquots of methanolic extracts were diluted with 50 ml distilled water and applied to an AflaTest-P immunoaffinity column (Vicam, Watertown, MA, USA) containing a monoclonal antibody specific for aflatoxins B₁, B₂, G₁, and G₂. The toxins were isolated, purified, and concentrated on the column, removed from antibodies with pure methanol, and quantified by reversed-phase HPLC with postcolumn derivatization and fluorescence detection, under the same conditions as described above.

### Inhibition test for Cholesterol synthesis :

The human hepatic T9A4 cells were grown in the serumfree LCM medium (Biofluids, Rockville, MD, USA) under 3.5% CO₂ at 37°C. The cells were seeded into 24-well plates and incubated at confluency with 1mM ¹⁴C-acetate (1 mCi/mmol, Amersham) for 20 h in the absence (control) or in the presence of the fermented extract fractions. Cells were also incubated with 1.17 mM ¹⁴C-mevalonate (0.85 mCi/mmol, Amersham) for 20 h in order to evaluate whether the compounds stemming from the micro-organisms to be tested have an inhibitory effect downstream the HMG-CoA -> Mevalonate conversion step.
Lipid extraction was performed twice by incubation with hexane:isopropanol (3:2) for 30 min at room temperature. The combined extracts were dried under N₂, redissolved in hexane and subjected to high performance thin layer chromatography (Merck, Darmstadt, Germany) in a solvent mixture of hexane:diethyl ether:acetic acid (75:25:1). The neo-synthesis of cholesterol was determined by measuring the ¹⁴C-acetate incorporation into the cholesterol with an instant imager (Camberra Packard, Zurich, Switzerland) and expressed as percent of the control.

Table 1 shows the *in vitro* hypocholesterolemic activity in the human hepatic T9A4 cells of the extracts in presence of ¹⁴-C acetate. It can easily be seen that the cholesterol synthesis in human hepatic T9A4 cells is reduced when the extracts obtained by fermentation with a micro-organism according to the invention are incubated with the cells. Since the lovastatin content in extract obtained from A4 an A27 strains is less than 0,1 µg/ml, the cholesterol synthesis inhibition effect observed can be attributed to lovastatin present in the extracts but also to other compounds similar to the ones that are produced by the other non-lovastatin producing strains.

The extracts obtained with strains A12, A21, A34, A39, A45, A50, A51, A53, FJ2 and FJ5 do not contain any trace of lovastatin (MS graph not shown) but has seen on Table 1, these extracts exhibit strong cholesterol biosynthesis inhibitory activity despite the lack of lovastatin. This indicates that such micro-organisms according to the invention produce compounds other than lovastatin capable of lowering serum cholesterol concentration that act as inhibitors of at least one step downstream of the step catalysed by HMG-CoA-reductase.

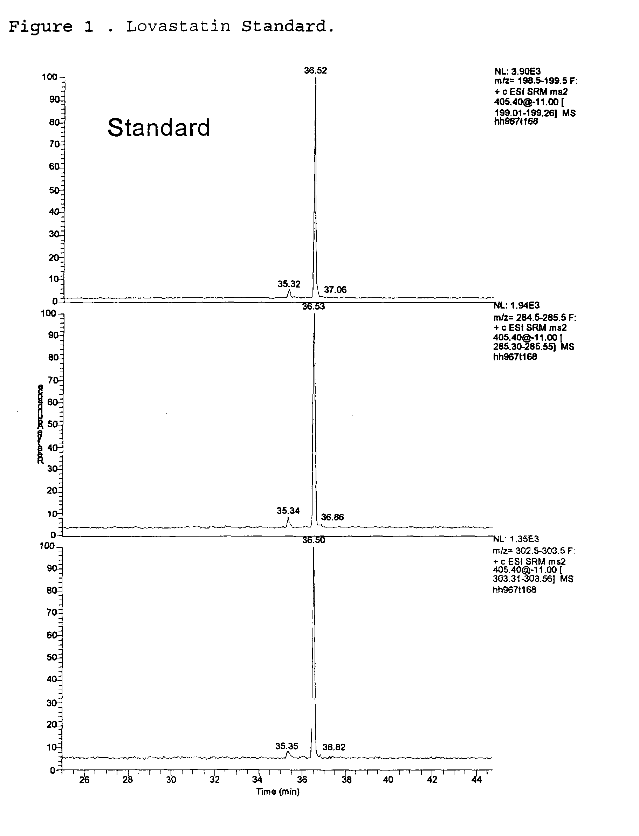

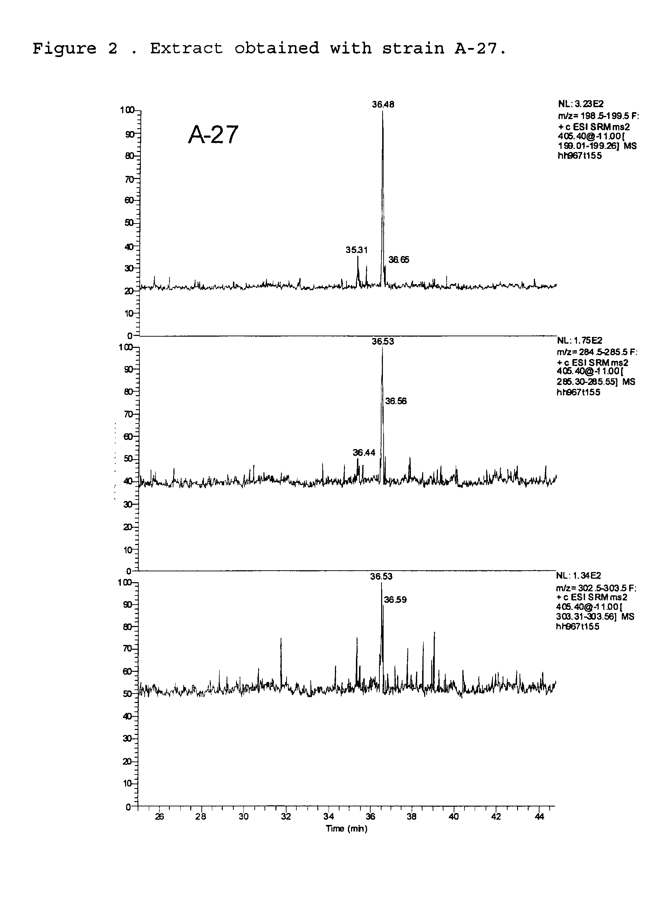

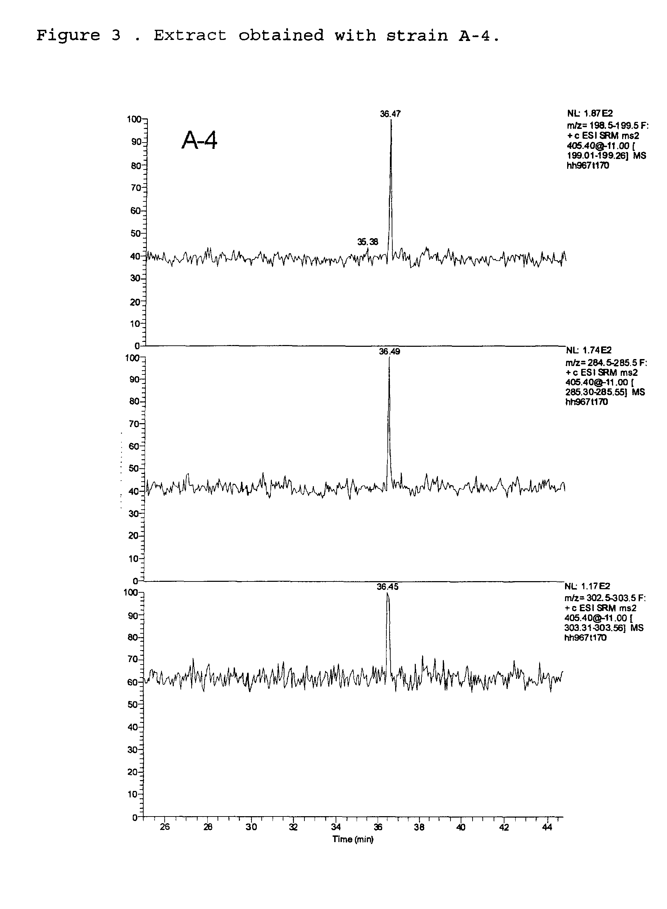

Figure 1 shows the MS graph obtained with lovastatin standard. Figure 2 and 3 show the MS graphs of the extracts obtained by fermentation with micro-organisms according to the invention, with strains A-27 and A-4, respectively. Lovastatin content of the extracts obtained with these strains is less than 0,1 µg/ml.
Of course none of the extracts obtained with the micro-organisms according to the invention contain any trace of aflatoxin.

Table 2 shows the *in vitro* cholesterol synthesis inhibiting activity in the human hepatic T9A4 cells of the extracts in presence ¹⁴C-mevalonate. In this case, it can be seen that the presence of lovastatin does not inhibit cholesterol biosynthesis even though the fermentation extracts obtained with the strains A21, A34, A39, FJ2 and FJ5 shows evidence of inhibitory effect on cholesterol biosynthesis. That means that these strains produce at least one compound that inhibits at least one step downstream of mevalonate.

**TABLE 1 :**

| The *in vitro* hypocholesterolemic activity in human hepatic assays of strains extracts in presence of ¹⁴C-acetate | | |
|---|---|---|
| Strain code | Final concentration of crude extract (µg dry matter /ml) | % inhibition cholesterol synthesis |
| | | |
| A4 | 39 | 23 |
| | | |
| A27 | 51 | 45 |
| | | |
| A12 | 70 | 59 |
| | | |
| A21 | 38 | 54 |
| | | |
| A34 | 50 | 55 |
| | | |
| A39 | 47 | 64 |
| | | |
| A45 | 44 | 47 |
| | | |
| A50 | 43 | 29 |
| | | |
| A51 | 41 | 7 |
| | | |
| A53 | 42 | 55 |
| | | |
| FJ2 | 37 | 61 |
| | | |
| FJ5 | 40 | 46 |
| | | |
| LOVASTATIN | 0,5 | 56 |

**TABLE 2 :**

| The *in vitro* hypocholesterolemic activity in human hepatic assays of strains extracts in presence of ¹⁴C-mevalonate | | |
|---|---|---|
| Strain code | Final concentration of crude extract (µg dry matter /ml) | % inhibition cholesterol synthesis |
| | | |
| A21 | 38 | 82 |
| | | |
| A34 | 50 | 66 |
| | | |
| A39 | 47 | 72 |
| | | |
| FJ2 | 37 | 64 |
| | | |
| FJ5 | 40 | 42 |
| | | |
| | | |
| LOVASTATIN | 0,5 | 0 |

### EXAMPLE 2

### Soy Koji fermentation

175 g of a mixture of defatted soybeans (75% w/w) and roasted wheat (25% w/w) were steam-cooked for 7 minutes at 120°C with closed lid in an autoclave and allowed to cool below 40°C. The cooked soybean-wheat mixture was inoculated with 10⁹ cfu of a conidiospore suspension of a micro-organism according to the invention (strain A27) in SP2 (20 mM KH₂PO₄-HCl pH 2.0, 0.9% NaCl). The inoculated mixture was transferred to a 3-liter mushroom spawn bag (porosity 25cc/min, filter with 160mm, Van Leer, United Kingdom) and incubated at 30°C in a Lab-Term incubator (Kühner, Switzerland) for up to 70 hours. Temperature inside the Koji bed was monitored not to exceed 34°C.

The obtained Koji is used for preparing an extract from liquid cell culture according to the method used in example 1.
It can easily seen on Table 3 that the extracts obtained from solid Koji fermentation exhibit cholesterol synthesis inhibition property.

### EXAMPLE 3

### Wheat bran Koji fermentation

The method is the same that the one of example 2 except the use of a mix of 120 g Wheat bran (dry matter : 86%) + 73 g Water + 100 µl Acetic acid, instead of a mix of soya beans and roasted wheat was used.
The extract is obtained by a method similar to the one described above.
It can easily seen on Table 3 that the extracts obtained from solid Koji fermentation exhibit cholesterol synthesis inhibition property.

**TABLE 3 :**

| The *in vitro* hypocholesterolemic activity in human hepatic assays of *strains* extracts in solid state in presence of ¹⁴C-acetate | | |
|---|---|---|
| Strain Code | Concentration µg/ml | % Inhibition Cholesterol synthesis |
| | | |
| **A27** ^{**a**}**(SK)** | **11** | **45** |
| | | |
| **A27** ^{**b**}**(WBK)** | **30** | **42** |

| | | |
|---|---|---|
| ^{a}K : Soya Koji | | |
| ^{b}WB : Wheat bran Koji | | |

## Claims

1. An isolated naturally occuring micro-organism wich is incapable of producing a toxin and wich produces at least one compound wich is capable of lowering serum cholesterol concentration.

2. A naturally occuring micro-organism wich is incapable of producing a toxin and wich produces at least one compound wich is capable of lowering serum cholesterol concentration for use in a fermented food product.

3. A micro-organism according to claim 1 or 2 **characterized in that** the cholesterol lowering effect is achieved by inhibiting cholesterol biosynthesis.

4. A micro-organism according to claim 1 to 3 chararacterized in that the cholesterol lowering effect is achieved by inhibiting HMG-CoA-reductase enzyme.

5. A micro-organism according to claim 1 to 4 **characterized in that** the compound wich is capable of lowering cholesterol concentration is lovastatin.

6. A micro-organism according claim 1 to 5 **characterized in that** the micro-organism is incapable of producing an aflatoxin.

7. A micro-organism according to claim 1 to 6 selected in the group consisting of the genus : Aspergillus, Monascus or Penicillium.

8. A micro-organism according to claim 1 or 2 **characterized in that** the compound wich is capable of lowering cholesterol concentration inhibits at least one step downstream of mevalonate.

9. Use of a micro-organism according to any preceding claim in the manufacture of a food product or a medicament for modulation of serum cholesterol levels or treatment of cholesterol related deseases.

10. Use of at least one compound stemming from a micro-organism according to claim 1 to 8 in the manufacture of a food product or a medicament for modulation of serum cholesterol levels or treatment of cholesterol related deseases.

11. Process for production of a fermented food product comprising the steps of :
- inoculating crop material with an inoculate comprising at least one micro-organism according claim 1 or 2 to effect fermentation,
- hydrolysing the resultant preparation to obtain an hydrolysate.

12. Food product with serum cholesterol-lowering properties **characterized in that** it contains at least one compound stemming from a micro-organism according to claims 1 to 8.
